(19) Europäisches Patentamt
European Patent Office
Office européen des brevets

(11) Veröffentlichungsnummer: **0 090 243**
**B1**

(12) # EUROPÄISCHE PATENTSCHRIFT

(45) Veröffentlichungstag der Patentschrift: **04.09.85**

(51) Int. Cl.⁴: **C 08 G 65/40,** C 07 C 43/263

(21) Anmeldenummer: **83102482.3**

(22) Anmeldetag: **14.03.83**

(54) **Verfahren zur Herstellung von aromatischen Äthern und aromatischen Polyäthern.**

(30) Priorität: **27.03.82 DE 3211421**

(43) Veröffentlichungstag der Anmeldung: **05.10.83 Patentblatt 83/40**

(45) Bekanntmachung des Hinweises auf die Patenterteilung: **04.09.85 Patentblatt 85/36**

(84) Benannte Vertragsstaaten: **DE FR GB IT**

(56) Entgegenhaltungen: **Keine Entgegenhaltungen**

(73) Patentinhaber: **BAYER AG, Konzernverwaltung RP Patentabteilung, D-5090 Leverkusen 1 Bayerwerk (DE)**

(72) Erfinder: **Bier, Gerhard, Dr., Rabenkopfstrasse 2 Wohnstift Freiburg App. 419, D-7800 Freiburg (DE)**
Erfinder: **Kricheldorf, Hans Rytger, Prof. Dr., Menzelstrasse 10, D-2000 Hamburg 52 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Es ist eine Reihe von Verfahren bekannt, aromatische Verbindungen über eine Ätherbrücke zu verbinden. Verfahren zur Herstellung aromatischer Polyäther wurden erst in jüngerer Zeit bekannt, vermutlich, weil bei der Polymerbildung nur hohe Ausbeuten der Verätherung zu den erforderlichen hohen Molekulargewichten führen. Während im niedermolekularen Gebiet Ausbeuten von 70 bis 80% befriedigen, sind solche Ausbeuten bei der Synthese von hochmolekularen Verbindungen nicht akzeptabel. Die Polyverätherung wird im allgemeinen durch Umsetzung eines aromatischen Halogenids bzw. Dihalogenids mit einem aromatischen Phenolat bzw. Diphenolat durchgeführt (vgl. US-PS Nrn. 4052365, 4169178, 4105635, GB-PS Nr. 1348630 und DE-PS Nr. 1545106). Dabei erfordert eine einigermassen vollständige Umsetzung die Verwendung hochsiedender Lösungsmittel wie Tetramethylsulfon, Nitrobenzol. Von Nachteil ist, dass die Diphenolate in der Regel ein anderes Lösungsverhalten haben als die Dihalogenide. Die Kondensationsreaktion wird gefördert, wenn das Halogenid bzw. Dihalogenid eine funktionelle Gruppe enthält, die das Halogen aktiviert. Solche funktionellen Gruppen sind die Sulfongruppe, die Carbonylgruppe, die Nitrogruppe in o- oder p-Substitution, beispielsweise bei der Umsetzung von p,p'-Dichlordiphenylsulfon mit dem Biskaliumsalz des Bisphenol-A. Die Vervollständigung dieser Umsetzung ist nach den DE-OS Nrn. 2330103 und 2705586 durch Verwendung von hochsiedenden polaren Dispergiermitteln wie dem cyclischen aromatischen Sulfonäther der Formel

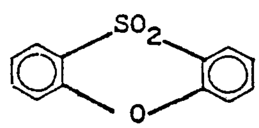

möglich.

Nach „J. Pol. Sc.", A1, Vol. 5, P. 2375 (1967), sind auch Difluorverbindungen als Dihalogenverbindung einsetzbar, wenn die Dihalogenverbindung die Struktur

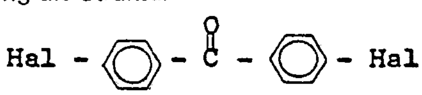

besitzt.

Bei der bekannten Polyverätherung fällt als Abspaltungsprodukt stets ein Salz, beispielsweise KCl oder KF an. Dieser Salzanfall erhöht aber die Viskosität der Mischung und erfordert umständliche Reinigungsverfahren. Die Verwendung von hochsiedenden Lösungsmitteln führt zu Lösungen der Polymeren, aus denen nur mit erheblichem Aufwand zur Entfernung von Hochsiedern und Aufarbeitung der grossen Lösungsmittelmengen die Polymeren isoliert und gereinigt werden können.

Da aromatische Polyäther eine hohe Thermostabilität und vorteilhafte physikalisch-chemische Eigenschaften besitzen, bestand die Aufgabe, ein besseres und einfacheres Verfahren zu deren Herstellung zu finden, das besonders die Kondensation und deren Vervollständigung erleichtert und keine schwer abtrennbaren Nebenprodukte entstehen lässt.

Gegenstand der Erfindung ist ein Verfahren zur Herstellung von aromatischen Äthern und aromatischen Polyäthern, welches dadurch gekennzeichnet ist, dass eine aromatische Fluorverbindung, worin ein oder mehrere Fluorsubstituenten an einem aromatischen Kern gebunden sind, mit einem Trialkylsilylderivat eines Phenols, worin eine oder mehrere Trialkylsilylgruppen an den Rest eines Mono- oder eines Polyphenols gebunden sind, bei erhöhter Temperatur unter Abspaltung von Trialkylfluorsilan umgesetzt wird.

Das erfindungsgemässe Verfahren hat den Vorteil, dass das entstehende flüchtige Trialkylfluorsilan bei der Reaktionstemperatur aus dem Reaktionsgefäss entweicht und der Polyäther nach vergleichsweise kurzer Reaktionszeit in sehr hoher Ausbeute und guter Reinheit vorliegt. Die Reaktion erfolgt nach dem Formelchema

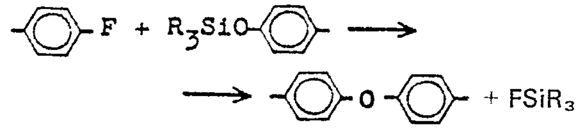

unter Austritt von Trialkylfluorsilan aus äquivalenten Mengen der Fluorsubstituenten der aromatischen Fluorverbindungen und der Trialkylsilylgruppen der Trialkylsilylderivate von Phenolen.

Entsprechend dem Reaktionschema können mehrere Gruppen von Ausgangsstoffen Verwendung finden, nämlich Monofluor-, Difluor- oder ggf. Polyfluorderivate aromatischer Verbindungen durch Umsetzung mit Mono-, Di- oder Poly-(trialkylsilyl)derivaten von Mono-, Di- oder Polyphenolen oder aromatische Verbindungen, die im selben Molekül einen oder mehrere Fluorsubstituenten und eine oder mehrere Trialkylsilylgruppen enthalten. Im Falle der Umsetzung von Monofluorderivaten aromatischer Verbindungen mit Trialkylderivaten von Monophenolen entstehen monomere Äther und bei Umsetzung von Difluorderivaten aromatischer Verbindung mit Trialkylsilylderivaten von Monophenolen im Molverhältnis 1:2 sowie bei Umsetzung von Monofluorderivaten aromatischer Verbindungen mit Bis(trialkylsilyl)-derivaten von Diphenolen entstehen Bisäther, deren Herstellung für bestimmte Zwecke bevorzugt ist.

Bei allen anderen Umsetzungen entstehen Polyäther, von denen die Herstellung linearer Polyäther durch Umsetzung von Difluorderivaten aromatischer Verbindungen mit Bis(trialkylsilyl)derivaten von Bisphenolen sowie ggf. durch Umsetzung gleicher oder verschiedener Monofluorderivaten von Trialkylsilylderivaten von Monophenolen bevorzugt ist.

Die Herstellung verzweigter oder vernetzter Polyäther durch Umsetzung von Trifluorderivaten aromatischer Verbindungen mit Tris-(trialkylsilyl)derivaten von Triphenolen bzw. der Zusatz solcher trifunktioneller Ausgangsstoffe zu den genannten di- oder monofunktionellen Ausgangsstoffen ist weniger bevorzugt.

Als Trialkylsilylderivate von Phenolen sind solche von 1 bis 10 Kohlenstoffatomen im Alkylrest möglich, die mit 1 bis 3 Kohlenstoffatomen im Alkylrest bevorzugt und die Trimethylsilylderivate sehr bevorzugt. Die Fluorderivate aromatischer Verbindungen und die Trialkylsilylderivate von Phenolen enthalten bevorzugt zwei aromatische Kerne im Molekül, obwohl Ausgangsstoffe mit einem aromatischen Kern möglich sind. Reaktionspartner bzw. Gemische von Ausgangsstoffen mit verschiedener Struktur der aromatischen Kerne sind zweckmässig.

Bevorzugt ist weiter, dass in Ausgangsstoffen mit zwei aromatischen Kernen die reaktiven Substituenten, nämlich die beiden Fluorsubstituenten, die beiden Trialkylsilylgruppen oder je ein Fluorsubstituent und eine Trialkylsilylgruppe oder je ein Fluorsubstituent und eine Trialkylsilylgruppe, an verschiedene der aromatischen Kerne gebunden sind. Weiter ist vielfach ein symmetrischer Bau der Moleküle bevorzugt, beispielsweise die Stellung der reaktiven Substituenten in 4,4'-Position der zweikernigen Aromaten und Diphenole, obgleich auch andere Positionen möglich sind.

Als aromatische Fluorverbindung sind solche besonders reaktiv bzw. geeignet und daher bevorzugt, die eine aktivierende Gruppe wie eine Sulfongruppe, Ketogruppe, $-CO_2R$-Gruppe, Phenylgruppe oder Nitrogruppe jeweils in o- oder p Stellung enthalten.

Beispiele für die Difluorverbindung:

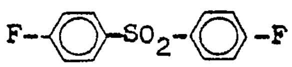

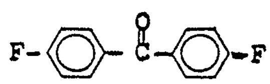

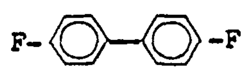

Beispiele für die Trialkyl-Si-Derivate von Phenolen und Diphenolen:

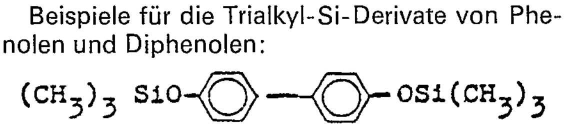

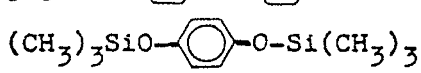

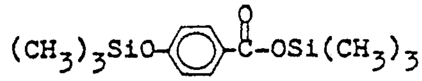

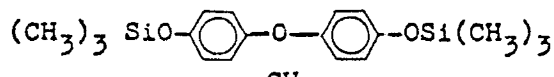

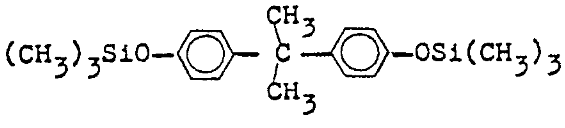

Es ist auch möglich, Verbindungen einzusetzen, die in einem Molekül die Fluor- und die Trialkyl-Si-Gruppe enthalten, z. B.:

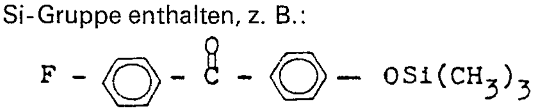

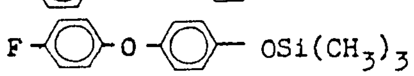

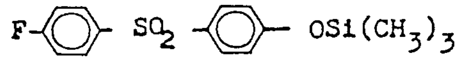

Die Fluorverbindungen sind in bekannter Weise aus den entsprechenden Aromaten zugängig. Die Trialkylsilylderivate von Phenolen sind einfach durch Umsetzung von Trialkylhalogensilan mit Alkaliphenolaten erhältlich, oder an den freien Phenolen mittels Trialkylchlorsilan/Triäthylamin sowie mittels Hexamethyldisilazan und anderer N-Trialkylsilyl-Stickstoffverbindungen. Fluorsubstituierte Phenole werden mittels Trialkylchlorsilan/Triäthylamin silyliert oder in Form der Alkalisalze mittels Trialkylchlorsilan.

Der besondere Vorteil des Verfahrens liegt in der einfachen Ausführbarkeit der Schmelzkondensation. Die Ausführung des Verfahrens als Schmelzkondensation in Abwesenheit von Lösungsmitteln ist daher entschieden bevorzugt. Die Kondensation in hochsiedenden Lösungsmitteln wie u. a. Nitrobenzol, Tetramethylsulfon ist ebenfalls möglich. Eine Reinigung der Polyäther ist im allgemeinen nicht erforderlich, jedoch kann eine Umfällung erfolgen, z. B. um physikalisch-chemische Messungen an reinsten Produkten durchzuführen.

Die Reaktionstemperatur liegt allgemein im Bereich zwischen 150 und 350° C, vorzugsweise zwischen 170 und 350° C. Dabei wird zweckmässig im Bereich von 170 bis 270° C zunächst Trialkylfluorsilan abgespalten und entfernt und darauf die Temperatur erhöht, um die Abspaltung von Trialkylfluorsilan und die Kondensation zu vervollständigen.

Überraschend werden sehr hohe Ausbeuten der Äther und Polyäther im Bereich von 96 bis über 98% und hohe Reinheiten erhalten. In Reaktionszeiten von nur 2,5 bis 3,5 h werden überraschend hochpolymere Polyäther erhalten. Der Druck kann zwischen 0,3 und etwa 2 bar (300-2000 kPa) liegen. Bevorzugt wird bei Normaldruck oder geringem Unterdruck gearbeitet. Das Reaktionsgefäss wird zweckmässig mit einer Vorrichtung zur Kondensation des Trialkylfluorsilans zu versehen, das erneut zur Umsetzung mit Alkaliphenolat verwendet wird.

Die Reaktion wird bevorzugt in Gegenwart eines Katalysators aus der Klasse der Fluoridsalze, wie Ammonfluorid oder einem der Alkalifluoride, besonders KF, RbF oder CsF, ausgeführt. Mengen von 0,1 bis 10 mg je Gramm der Ausgangsstoffe sind zweckmässig.

Es ist möglich, den Kondensationsgrad durch Wahl der Ausgangsstoffe und der Reaktionsbedingungen in sehr weiten Grenzen zu beeinflussen. Beispielsweise kann zur Herstellung von oligomeren Verbindungen die Polykondensation bei der Erreichnung bestimmter mittlerer Kondensationsgrade unterbrochen werden und ggf. ein Verschluss der Endgruppen mit Hilfe berechneter Mengen von Monofluorderivaten aromatischer Verbindungen oder Trialkylsilylderivaten von Monophenolen erfolgen. Das Verfahren ist besonders zur Herstellung von Blockpolymeren geeignet, in denen die einzelnen Blöcke auf Monomere verschiedener Struktur zurückgehen. Auf diese

Weise kann man z. B. Polyarylester oder Polyarylketone mit Polyaryläthern verbinden, Polyaryläther von verschiedener Struktur der aromatischen Kerne verknüpfen oder sich wiederholende Abschnitte von Polyarylsulfonen und Polyaryläthern aneinanderfügen. Durch weitere Umsetzung mit trifunktionellen Ausgangsstoffen ist die weitere Erhöhung des Molekulargewichts bzw. die Vernetzung oder Härtung von Oligomeren oder Hochpolymeren möglich.

Die Produkte können auf dem Lacksektor, für Beschichtungen, besonders aber als Hochpolymere zur Herstellung von Fasern, Fäden und Formkörpern dienen.

Die Produkte weisen eine hohe Thermostabilität auf. An getrockneten Proben der Beispiele 1 und 2 wurde durch thermographische Analyse mit einer Heizrate von 8° K/min festgestellt, dass bis 380° C kein Gewichtsverlust auftritt, bis 430° C 1 Gew.-% und bis 444° C 2 Gew.-% Gewichtsverlust erfolgt. Bis etwa 450° C sind die Proben thermisch stabil. Erst ab etwa 560° C erfolgt eine exotherme Zersetzung.

*Beispiel 1:*

37,1 g (0,1 mol) Bistrimethylsilyl-Bisphenol-A und 25,4 g (0,1 mol) 4,4'-Bisfluorphenylsulfon werden im schwachen $N_2$-Strom zusammen mit 50 mg Kaliumfluorid auf 270° C erhitzt, wobei im Lauf von 10 min eine kräftige Abspaltung von Trimethylfluorsilan einsetzt. Die nach 30 min viskos gewordene Schmelze wird noch für 2,5 h auf 320° C erhitzt. Dabei kommt die Abspaltung von Fluortrimethylsilan zum Erliegen und aus der hochviskosen Schmelze lassen sich Fäden ziehen. Durch Lösen in Methylenchlorid und Fällen mit Methanol wird ein weisses, fasriges Polymere erhalten (97% Ausbeute), das bei der dampfdruckosmotischen Bestimmung des Molekulargewichts ein Zahlenmittel von 8500-9500 ergibt. Mit C = 2 g/l wird in Tetrachloräthan bei 20° C $\eta_{inh}$ = 0,7-0,8 dl/g gefunden.

*Beispiel 2:*

37,1 g (0,1 mol) Bistrimethylsilyl-Bisphenol-A und 25,4 g (0,1 mol) 4,4'-Bisfluorphenylsulfon werden mit 50 mg Caesiumfluorid im schwachen $N_2$-Strom auf 170° C erhitzt, wobei eine kräftige Abspaltung von Trimethylfluorsilan einsetzt. Nach 30 min wird die Temperatur für 2,5 h auf 320° C erhöht und das Polymere aus einer Lösung von Methylenchlorid mit Methanol gefällt. Die Ausbeute an weissem Polyäther beträgt 98%. Die inherente Viskosität, gemessen in Tetrachloräthan mit C = 2 g/l, liegt bei 1,4 dl/g.

*Beispiel 3:*

39,4 g (0,1 mol) Bistrimethylsilyl-4,4'-dihydroxydiphenylsulphon, 25,4 g (0,1 mol) 4,4'-Bisfluorphenylsulfon und 50 mg Caesiumfluorid werden auf 270° C erwärmt, worauf eine kräftige Abspaltung von Trimethylfluorsilan einsetzt. Nach 30 min wird das Reaktionsgemisch für 2,5 h auf 340° C erhitzt und das Produkt aus einer Lösung von Tetrachloräthan mit Methanol gefällt:

Ausbeute: 97% eines weissen, fasrigen Polymeren. Die Dampfdruckosmose in Tetrachloräthan ergab Mn = 16 000.

*Beispiel 4:*

28,2 g (0,1 mol) Bistrimethylsilyl-4-hydroxybenzoesäure, 25,4 g (0,1 mol) 4,4'-Bisfluorphenylsulfon und 50 mg Caesiumfluorid werden auf 270° C erhitzt, wobei Trimethylfluorsilan abgespalten wird. Nach 1 h wird die Schmelze auf 320° C erhitzt und nach 2 h aus Tetrachloräthan/Methanol gefällt:

Ausbeute: 96% eines gelblichen fasrigen Polymeren.

*Beispiel 5:*

37,1 g (0,1 mol) Bistrimethylsilyl-Bisphenol-A, 21,8 g (0,1 mol) 4,4'-Difluorbenzophenon und 50 mg Caesiumfluorid werden 1 h auf 300° C erhitzt, wobei unter Abspaltung von Trimethylfluorsilan eine viskose Schmelze entsteht, die noch 2 h bei 320° C nachkondensiert wird. Nach Umfällen aus Tetrachloräthan/Methanol wird ein weisses, fasriges Produkt in einer Ausbeute von 96% erhalten.

*Beispiel 6:*

33,0 g (0,1 mol) Bistrimethylsilyl-4,4'-dihydroxydiphenyl, 21,8 g (0,1 mol) 4,4'-Difluorbenzophenon und 50 mg CsF werden im schwachen Stickstoffstrom auf 300° C erhitzt, wobei die Abspaltung von Trimethylfluorsilan einsetzt. Nach 2 h wird die Temperatur für weitere 2 h auf 330° C gesteigert, wobei der Kolbeninhalt erstarrte. Da das Polyätherketon bis 420° C keinen Schmelzpunkt aufweist und sich bei höheren Temperaturen zersetzt, wird es in der Kälte mechanisch zerkleinert und mit siedendem Methylenchlorid gewaschen. Das hellbraune Produkt wurde in einer Ausbeute von 94% erhalten.

Das Produkt ist löslich u. a. in Methylenchlorid, Tetrachloräthan, Dimethylsulfoxid, Dimethylformamid-5% LiCl und Trifluoressigsäure.

Herstellung der Ausgangsstoffe:

*Beispiel A:*

228 g (1 mol) Bisphenol-A und 400 g (ca. 2,5 mol) Hexamethyldisilazan werden zum Rückfluss erhitzt bis die Entwicklung von Ammoniak zum Erliegen kommt. Dann wird das überschüssige Hexamethyldisilazan im Vakuum entfernt, und das Bistrimethylsilyl-Bisphenol-A durch Destillation im Vakuum eingeengt. Ausbeute: 98%.

*Beispiel B:*

228 g (1 mol) Bisphenol-A und 220 g (2 mol) Trimethylchlorsilan werden in 2,5 l trockenem Toluol gelöst und unter kräftigem Rühren in der Wärme 209 g (2 mol) Triäthylamin zugetropft. Danach wird das Reaktionsgemisch 20 min zum Rückfluss erhitzt, auf 10-20° C abgekühlt, rasch filtriert und das Bistrimethylsilyl-Bisphenol-A aus dem Filtrat durch Destillation im Vakuum isoliert. Ausbeute: 91%.

*Beispiel C:*

119 g (0,5 mol) Natriumsalz des 4-Fluor-4'-hydroxybenzphenols werden in 500 ml trockenem Tetrahydrofuran (oder Dioxan, Acetonitril) suspendiert und zum Rückfluss erhitzt. Dann wird unter Rühren rasch 0,5 mol Trimethylchlorsilan zugetropft, dann das Reaktionsgemisch noch 20 min zum Rückfluss erhitzt. Das Lösungsmittel wird abdestilliert und das Produkt durch Destillation im Vakuum isoliert. Ausbeute: 91%.

## Patentansprüche

1. Verfahren zur Herstellung von aromatischen Äthern und aromatischen Polyäthern, dadurch gekennzeichnet, dass eine aromatische Fluorverbindung, worin ein oder mehrere Fluorsubstituenten an einem aromatischen Kern gebunden sind, mit einem Trialkylsilylderivat eines Phenols, worin eine oder mehrere Trialkylsilylgruppen an den Rest eines Mono- oder eines Polyphenols gebunden sind, bei erhöhter Temperatur unter Abspaltung von Trialkylfluorsilan umgesetzt wird.

2. Verfahren nach Anspruch 1, dadurch gekennzeichnet, dass als Trialkylsilylderivat eines Phenols das Trimethylsilylderivat eingesetzt wird.

3. Verfahren nach einem der Ansprüche 1 oder 2, dadurch gekennzeichnet, dass áls aromatische Fluorverbindung eine aromatische Difluorverbindung und als Trialkylsilylderivat eines Phenols ein Bistrialkylsilylderivat eines Diphenols eingesetzt wird.

4. Verfahren nach den Ansprüchen 1 bis 3, dadurch gekennzeichnet, dass in der aromatischen Fluorverbindung das F durch eine o- oder p-ständige negativierende Gruppe, wie Sulfo, Nitro, Keto, Phenyl oder −COOR aktiviert ist.

5. Verfahren nach einem der Ansprüche 1 oder 2, dadurch gekennzeichnet, dass das Trialkylsilylderivat eines Fluorphenols eingesetzt wird.

6. Verfahren nach mindestens einem der Ansprüche 1 bis 5, dadurch gekennzeichnet, dass die Fluorverbindungen und Trialkylsilylderivate eines Phenols zwei oder mehr aromatische Kerne enthalten.

7. Verfahren nach Anspruch 6, dadurch gekennzeichnet, dass die Fluorsubstituenten und/oder Trialkylsilylgruppen an verschiedene der aromatischen Kerne gebunden sind.

8. Verfahren nach mindestens einem der Ansprüche 1 bis 7, dadurch gekennzeichnet, dass eine katalytisch wirkende anorganische Fluorverbindung vom Typ der Ammoniumfluoride oder Alkalifluoride, vorzugsweise Kalium-, Rubidium- oder Caesiumfluorid, bevorzugt in Mengen von 0,1 bis 10 mg je Gramm der Ausgangsstoffe, zugegen ist.

9. Verfahren nach mindestens einem der Ansprüche 1 bis 8, dadurch gekennzeichnet, dass die Kondensation in Schmelze durchgeführt wird.

10. Verfahren nach mindestens einem der Ansprüche 1 bis 9, dadurch gekennzeichnet, dass die Kondensation in Gegenwart eines hochsiedenden Lösungsmittels durchgeführt wird.

## Claims

1. Process for the production of aromatic ethers and aromatic polyethers, characterised in that an aromatic fluorine compound, wherein one or more fluorine substituents are attached to an aromatic nucleus, is reacted with a trialkylsilyl derivative of a phenol, wherein one or more trialkylsilyl groups are attached to the radical of a mono- or polyphenol, at elevated temperature, trialkylfluorosilane being split off.

2. Process according to Claim 1, characterised in that the trimethylsilyl derivative is used as the trialkylsilyl derivative of a phenol.

3. Process according to one of Claims 1 or 2, characterised in that an aromatic difluorine compound is used as the aromatic fluorine compound and a bis-trialkylsilyl derivative of a diphenol is used as the tiralkylsilyl derivative of a phenol.

4. Process according to Claims 1 to 3, characterised in that, in the aromatic fluorine compound, the F is activated by an o- or p-position group imparting electronegativity, such as sulpho, nitro, keto, phenyl or −COOR.

5. Process according to one of Claims 1 or 2, characterised in that the trialkylsilyl derivative of a fluorophenol is used.

6. Process according to at least one of Claims 1 to 5, characterised in that the fluorine compounds and trialkylsilyl derivatives of a phenol contain two or more aromatic nuclei.

7. Process according to Claim 6, characterised in that the fluorine substituents and/or trialkylsilyl groups are attached to different aromatic nuclei.

8. Process according to at least one of Claims 1 to 7, characterised in that a catalytically acting inorganic fluorine compound of the ammonium fluoride or alkali fluoride type, preferably potassium, rubidium or caesium fluoride, is present, preferably in amounts of 0.1 to 10 mg/g of the starting materials.

9. Process according to at least one of Claims 1 to 8, characterised in that the condensation is carried out in the melt.

10. Process according to at least one of Claims 1 to 9, characterised in that the condensation is carried out in the presence of a high-boiling solvent.

## Revendications

1. Procédé de préparation d'éthers aromatiques et de polyéthers aromatiques, caractérisé en ce que l'on fait réagir un composé aromatique fluoré dans lequel un ou plusieurs substituants fluoro sont fixés sur un noyau aromatique, avec un dérivé trialkylsilylé d'un phénol dans lequel un ou plusieurs groupes trialkylsilyle sont fixés sur le reste d'un mono- ou un polyphénol, à température élevée, avec scission de trialkylfluorsilane.

2. Procédé selon la revendication 1, caractérisé

en ce que l'on utilise en tant que dérivé trialkylsilylé d'un phénol le dérivé triméthylsilylé.

3. Procédé selon l'une des revendications 1 ou 2, caractérisé en ce que l'on utilise en tant que composé aromatique fluoré un composé aromatique difluoré et en tant que dérivé trialkylsilylé d'un phénol un dérivé bis-trialkylsilylé d'un diphénol.

4. Procédé selon les revendications 1 à 3, caractérisé en ce que, dans le composé aromatique fluoré, le fluore est activé par un groupe négativant en position o- ou p-, par exemple un groupe sulfo, nitro, céto, phényle ou $-COOR$.

5. Procédé selon l'une des revendications 1 ou 2, caractérisé en ce que l'on utilise le dérivé trialkylsilylé d'un fluorophénol.

6. Procédé selon au moins une des revendications 1 à 5, caractérisé en ce que les dérivés fluorés et les dérivés trialkylsilylés d'un phénol contiennent deux ou plusieurs noyaux aromatiques.

7. Procédé selon la revendication 6, caractérisé en ce que les substituants fluoro et/ou les groupes trialkylsilyle sont fixés sur des noyaux aromatiques différents.

8. Procédé selon au moins une des revendications 1 à 7, caractérisé en ce que l'on opère en présence d'un composé fluoré minéral ayant un effet catalytique, du type des fluorures d'ammonium ou des fluorures alcalins, de préférence le fluorure de potassium, le fluorure de rubidium ou le fluorure de caesium, de préférence en quantité de 0,1 à 10 mg/g des produits de départ.

9. Procédé selon au moins une des revendications 1 à 8, caractérisé en ce que la condensation est effectuée à l'état fondu.

10. Procédé selon au moins une des revendications 1 à 9, caractérisé en ce que la condensation est effectuée en présence d'un solvant à haut point d'ébullition.